# EUROPEAN PATENT SPECIFICATION

(11) **EP 0 594 099 B1**
(45) Date of publication and mention of the grant of the patent: **24.06.1998**
(21) Application number: 93116779.5
(22) Date of filing: 18.10.1993
(51) Int. Cl.: C07D 503/18

(54) **Process for the purification of crude clavulanic acid**
Verfahren zur Reinigung von roher Clavulansäure
Procédé pour la purification d'acide clavulanique cru

(30) Priority: 21.10.1992 KR 9219353
(43) Date of publication of application: 27.04.1994
(73) Proprietor: PHARMA DEVELOPMENT LTD., Dublin 2 (IE)
(72) Inventor: Yang, Ho Suk, Kangnam-Gu, Seoul (KR); Choi, Nam Hee, Kangnam-Gu, Seoul (KR); Lee, Sang Chul, Pundang-Gu, Sungnam-Si, Kyeonggi-Do (KR); Ham, Yun Beom, Nam-Gu, Inchon (KR); Min, Kyeong Bok, Kwanak-gu, Seoul (KR)
(74) Representative: Giambrocono, Alfonso, Dr. Ing.

(56) References cited:
- EP-A- 0 026 044
- EP-A- 0 672 669
- BE-A- 862 211
- GB-A- 1 508 977
- GB-A- 1 543 563
- GB-A- 2 264 944
- J.C.S.Chem.Comm., 1979, p.282, D.Brown and J.R.Evans
- Federal Register, vol.50, no.161,1985, p.33519

## Description

The present invention relates to a method for the purification of crude clavulanic acid of formula: (1) 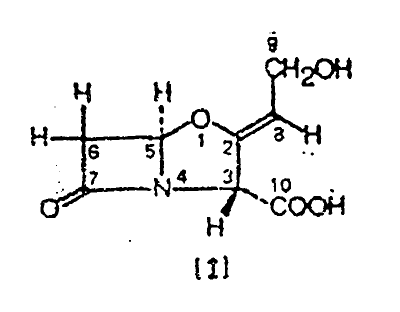 and its derivatives.

The GB-A- 1.508.977 discloses inter alia that clavulanic acid and its salts can be produced by the submerged fermentation of a number of streptomyces micro-organisms, for example Streptomyces clavuligerus, Streptomyces jumonjinensis and Streptomyces katsurahamanus.

A number of methods for recovering clavulanic acid from fermentation broths have been described, for example in the above mentioned British Patent.

These methods have utilized a variety of standard techniques such as ion exchange chromatography and solvent extraction of an aqueous solution of impure clavulanic acid.

Quite often such methods result in a product of low general purity containing small amounts of toxic contaminants.

As it is well known, for pharmaceutical use highly purified non-toxic clavulanic acid or its derivatives are required.

For instance, one method for preparing purified clavulanic acid as its potassium salt via a tertiary butylamine (TBA) salt intermediate has been described in European Patent EP-B 0026044. In particular, the impure clavulanic acid has to be extracted into ethyl acetate subsequently to which is added an equal volume of acetone as co-solvent and then TBA is added which results in the crystallization of TBA salt of clavulanic acid which is isolated as an intermediate.

This method requires the use of a large volume of solvent mixture and furthermore, in the presence of acetone, the TBA salt forms a solvate with acetone of variable composition. The content of acetone in the solvate may vary between 2% and 8%.

Also it is an important fact that TBA (the boiling point of which is 44.4°C) is miscible with water in all proportions.

Such method suffers from serious drawbacks deriving from the use of tertiary butylamine which is highly toxic (Oral LD (rat):180 mg/kg. See: "Dangerous Properties of Industrial materials", fifth edition, N. Irving Sax) and volatile (boiling point 44.4°C at 760 mmHg, see: "Chemical Rubber Company Handbook of Chemistry and Physics", 53rd edition 1972-73, page C-400).

As a result the use for tertiary butylamine on an industrial scale can be unduly hazardous and potentially dangerous for operating personnel.

Moreover, because tertiary butylamine is soluble in water in all proportions (C.R.C. Handbook as above) it is therefore difficult to recover from aqueous waste streams after removal of solvent.

This can result in adverse process economics but, more important, can result in a potential pollution problem associated with the discharge of tertiary butylamine in factory effluent. Also the use of large amounts of solvents mixtures, as disclosed in EP 0026044, can result in additional unfavourable process economics arising from the need to recover pure solvents from these mixtures.

Accordingly, the use of alternative amines has been investigated.

A number of amine salts of clavulanic acid have been described previously, e.g. in GB-A-1508977, BE-A- 862211 and GB-A-1543563.

From the literature data it appears that amine salts of clavulanic acid are formed with secondary or tertiary amines or with primary amines, the side chain of which contains secondary or tertiary alkyl units.

Many of such amines are either unsuitable for the production of a salt of clavulanic acid or they give rise to amine salts of clavulanic acid which are either hygroscopic or toxic or both and, therefore, are unsuitable for use as intermediates for the preparation of a pharmaceutically acceptable compound.

Experimental tests have now been carried out to find an amine which would be useful for the production of an amine salt of clavulanic acid which could be isolated as an intermediate for the eventual production of non-toxic clavulanic acid and its pharmaceutically acceptable salts, e.g. the potassium salt.

Almost all the salts derived from a wide range of primary, secondary and tertiary amines were found to be unsuitable for this purpose.

In particular a large number of primary and secondary amines having structures similar or close to those of the amines cited in the above mentioned references have been investigated but with unacceptable results.

Now it has been surprisingly found that tertiary octyl amine (2,4,4-trimethyl-2-pentanamine or TOA) is suitable for the production of a highly pure crystalline salt of clavulanic acid which may readily be isolated as a stable intermediate. This intermediate salt may then be used for the preparation of a highly pure non-toxic clavulanic acid and its pharmaceutically acceptable salts.

Therefore the present invention relates to a process for the preparation of substantially pure clavulanic acid as its pharmaceutically acceptable salts characterized in that an aqueous solution of impure clavulanate at a temperature between 0 and 5°C is acidified and the clavulanic acid extracted into a water immiscible organic solvent, expecially ethylacetate, to give a solution of clavulanic acid in an organic solvent which is subsequently dried and decolorized according to the usual techniques; to the dried decolorized organic solvent solution of clavulanic acid at 5°C tertiary octyl amine or a solvent solution thereof is added so as to cause the formation of substantially pure crystalline tertiary octyl amine salt of clavulanic acid which is recovered by usual techniques; the thus prepared intermediate tertiary octyl amine salt of clavulanic acid is dissolved in isopropanol containing an amount of water comprised between 0 and 5% to which is added an alcohol solution of potassium ethyl hexanoate such that the desired pharmaceutically acceptable crystalline salt of clavulanic acid is obtained and then isolated by the usual techniques.

The extraction of impure clavulanic acid is performed at temperatures of less than 10°C and, preferably, less than 5°C in water immiscible organic solvents, especially esters and ketones and more especially ethylacetate.

The drying is performed with a suitable desiccant, particularly magnesium sulphate.

The decolorization is carried out by usual decolorating agents, preferably with active charcoal. The subsequent addition of tertiary octyl amine or of a solution thereof to the dried decolorized organic solvent solution of clavulanic acid is performed at temperatures of less than 10°C and, preferably, less than 5°C.

The tertiary octyl amine salt of clavulanic acid is then dissolved in an organic solvent, especially an alcohol and more especially isopropanol containing an amount of water comprised between 0 and 5% and more especially between 0 and 2%.

To this solution, a solution of a suitable cation carrier, e.g. potassium 2-ethylhexanoate (KEH) in isopropanol is added such that potasium clavulanate is formed by a process of metathesis and caused to crystallize and then isolated according to usual techniques.

Of course it is to be understood that carriers of other ions which lead to pharmaceutically acceptable salts of clavulanic acid are to be included within the scope of this invention. As ion carriers, within the scope of this invention, are to be intended all the compounds capable of supplying the necessary cations.

Also, whilst isopropanol is known to be a suitable solvent for this purpose, it will be obvious to those skilled in the art that other similar solvents or mixtures of solvents may be equally useful and are also to be included within the scope of this invention.

By the use of TOA, it is possible to precipitate the TOA salt of clavulanic acid directly form ethylacetate without the use of any co-solvent (while the corresponding use of TBA to form the TBA salt of clavulanic acid requires the addition of an equal volume of acetone: see EP-B-0026044); this effords an important advantage since substantially less solvent is required and the recovery process is much simplified, thus improving the economics of the recovery process.

It has been further found that the TOA salt of clavulanic acid obtained by the method above disclosed is practically free from clavam-2-carboxylate: to the point that this impurity is practically undetectable by HPLC in the eventual potassium clavulanate. This is very surprising in that it is known that clavulanic acid is difficult to separate from clavam-2-carboxylic acid (J.C.S. Chem. Comm. 1979 page 282 D. Brown, J.R. Evans, R.A. Fletton "Structures of three novel beta-lactams isolated from Steptomyces clavuligerus").

Furthermore the use of TOA affords also the advantage that it is possible to precipitate the TOA salt of clavulanic acid from very dilute solutions; for instance the addition of 2 equivalents of TOA to a solution of 2000 g/ml of clavulanic acid in ethyl acetate gave a crystalline precipitate.

This feature is very important because it permits the direct solvent extraction of relatively dilute aqueous solutions of impure clavulanic acid; such as those which might be obtained as ion exchange column eluate.

In contrast to the TBA salt of clavulanic acid, it has been found that the crystalline TOA salt of clavulanic acid shows no evidence of solvate formation (the TBA salt forms the solvate with acetone with a composition variable between 2% and 8%: see EP-B-0026044). This is highly advantageous since the TOA salt is a homogeneous product which can be assayed on a direct clavulanic acid content basis (without the necessity of having to take account of variable amounts of solvent).

Furthermore the TOA is only very slightly soluble in water (while TBA is miscible with water in all proportions) which makes it easier to recover the TOA from process waste streams. This is important for process economics and for avoidance of pollution in the factory effluent.

The TOA salt per se is already known and has been described in BE-A-862211 but only as a suitable ingredient for pharmaceutical formulations: furthermore the process used to prepare the TOA salt of clavulanic acid in BE-A-862211 was not the same as that described herein.

The starting material in BE-A-862211 was an already purified benzylester derivative of clavulanic acid whereas the process described herein uses an aqueous solution of impure clavulanate.

The benzylester was converted to clavulanic acid via a potentially hazardous hydrogenation reaction which is totally avoided in the present process.

Furthermore the solvent used was tetrahydrofuran previously dried by distillation over calcium hydride which also is potentially hazardous and dangerous. In contrast, the present process uses ethylacetate in a much safer aqueous environment.

The features of the process described herein render this process much more suitable for industrial scale production.

### EXAMPLE 1

A solution of 0.8 g of impure potassium clavulanate in 20 ml of 1M sodium chloride was chilled to 5°C.

Ethyl acetate (20 ml) previously chilled to 5°C was then added and the mixture stirred vigorously. Sulfuric acid 25% v/v was then added dropwise to pH 2.0.

The ethyl acetate was separated and the remaining aqueous solution was extracted with three further 20 ml aliquots of cold ethyl acetate.

The ethyl acetate extracts were combined. Anhydrous magnesium sulfate (4g) was added and the mixture stirred at 5°C for 15 minutes.

There was then added decolorising charcoal 0.8g and the mixture stirred for a further 15 minutes. The magnesium sulfate and the decolorising charcoal were then removed by filtration.

Any residual product was washed through with ethylacetate 5ml at 5°C. A solution of tertiary octyl amine 1g in ethylacetate 5 ml was added dropwise over 30 minutes.

The resulting crystalline slurry was stirred for 2 hours at 5°C. The product was filtered, washed with a little ethyl acetate and dried in vacuo. The product which was a white crystalline solid weighed 1.05 g (yield 82%).

### EXAMPLE 2

An aqueous solution (500 ml) containing clavulanic acid (12 mg/ml) was chilled to 5°C.

Ethyl acetate (1,000 ml) previously chilled to 5°C was then added, and the mixture was stirred vigorously. Sulfuric acid 25% (v/v) was then added dropwise to pH 1.5.

The ethyl acetate was separated and the remaining aqueous solution was re-extracted with a furhter 1,000 ml aliquot of cold ethyl acetate.

The ethyl acetate extracts were combined. Anhydrous magnesium sulfate (50 g) and activated carbon (6 g) were added, and the mixture was then stirred at 5°C for 15 minutes.

The magnesium sulfate and activated carbon were removed by filtration.

Any residual product was washed by 125 ml of ethyl acetate with stirring at 5°C. A solution of tertiary octyl amine (6g) in ethyl acetate (30 ml) was added over 30 minutes.

The resulting crystalline slurry was stirred for 2 hours at 5°C. The product was filtered, washed with a little cold acetone and dried in vacuo.

The product which was a white crystalline solid weighed 8.12 g (Assay 58.0% free acid. Yield, corrected for purity 78.5%).

### EXAMPLE 3

Isopropanol (16 ml) and water (4 ml) was added to the product of Example 2, tertiary octyl amine salt (8.12 g), and the mixture was then stirred at room temperature to be dissolved.

The solution was filtered by suction through a filter with nitrogen purge, washed with isopropanol (20 ml).

Isopropanol (150 ml) was added to the combined filtrate and washing.

Potassium ethyl hexanoate in isopropanol (17.3 ml of 2N solution, 1.4 equivalents) was added dropwise at room temperature, and the resulting mixture was stirred for 2 hours and then chilled at 0 - 5°C.

The product was filtered by suction through a filter with nitrogen purge, washed with could acetone (20 ml each) three times.

Then the product was dried under vacuum at room temperature for 8 hours to yield potassium clavulanate (4.7 g).
- (Analysis:: purity; 82.5%
residual solvent; IPA 0,28%, Acetone 0.24%
moisture content; 0.75%
pH; 7.5
Clavam-2-carboxylate; < 0.005%
Yield, corrected for purity; 82.3%)

### COMPARATIVE EXAMPLE 1

An aqueous solution (300 ml) containing clavulanic acid (10.5 mg/ml) was treated as Example 2 to obtain the ethyl acetate extract (1200 ml). To the extract, was added anhydrous magnesium sulfate and the mixture stirred for 10 minutes. Decolorising charcoal (30 g) was then added and the mixture was stirred for further 10 minutes at 5°C.

The magnesium sulfate and decolorising charcoal were removed by filtration. The filter residue was washed with ethyl acetate (100 ml) and the washings combined with the rich filtrate.

The solution of n-hexylamine (2.15 ml) dissolved in acetone (10 ml) was then added slowly over 30 minutes. The mixture was stirred for further 2 hours at 5°C.

n-Hexylamine clavlunate did not come out.

## Claims

1. A process for the preparation of substantially pure clavulanic acid as its pharmaceutically acceptable salts characterized in that an aqueous solution of impure clavulanate at a temperature between 0 and 5°C is acidified and the clavulanic acid extracted into a water immiscible organic solvent, especially ethylacetate, to give a solution of clavulanic acid in an organic solvent which is subsequently dried and decolorized according to the usual techniques; to the dried decolorized organic solvent solution of clavulanic acid at 5°C, tertiary octyl amine or a solvent solution thereof is added so as to cause the formation of substantially pure crystalline tertiary octyl amine salt of clavulanic acid which is recovered by the usual techniques; the thus prepared intermediate tertiary octyl amine salt of clavulanic acid is dissolved in isopropanol to which is added an alcohol solution of potassium ethyl hexanoate such that the desired pharmaceutically acceptable crystalline salt of clavulanic acid is obtained and then isolated by the usual techniques.

2. A process as described in claim 1, in which the tertiary octylamine salt of clavulanic acid is formed and used as an intermediate in the preparation of pharmaceutically acceptable potassium clavulanate.

## Patentansprüche

1. Verfahren zur Herstellung von im wesentlichen reiner Clavulansäure in Form ihres pharmazeutisch annehmbaren Salzes, dadurch **gekennzeichnet,** daß eine wäßrige Lösung unreinen Clavulanates bei einer Temperatur zwischen 0 und 5°C angesäuert wird und die Clavulansäure in ein mit Wasser nicht mischbares organisches Lösungsmittel, insbesondere Ethylacetat, extrahiert wird, um eine Lösung von Clavulansäure in einem organischen Lösungsmittel zu ergeben, die dann getrocknet und gemäß üblicher Verfahren entfärbt wird; daß zu der getrockneten entfärbten organischen Lösungsmittellösung der Clavulansäure bei 5°C tertiäres Octylamin oder eine Lösungsmittellösung davon so zugegeben wird, daR die Bildung eines im wesentlichen reinen kristallinen tertiären Octylaminsalzes der Clavulansäure erfolgt, das durch übliche Verfahren gewonnen wird; daß das so hergestellte tertiäre Octylaminsalz der Clavulansäure als Zwischenprodukt in Isopropanol gelöst wird, zu welchem eine Alkohollösung von Kaliumethylhexanoat zugegeben wird, so daß das gewünschte pharmazeutisch annehmbare kristalline Salz der Clavulansäure erhalten und dann mittels üblicher Verfahren isoliert wird.

2. Verfahren nach Anspruch 1, dadurch **gekennzeichnet,** daß das tertiäre Octylaminsalz der Clavulansäure gebildet und als Zwischenprodukt bei der Herstellung pharmazeutisch annehmbaren Kaliumclavulanates verwendet wird.

## Revendications

1. Procédé pour préparer de l'acide clavulanique essentiellement pur, sous forme de ses sels acceptables d'un point de vue pharmaceutique, caractérisé en ce qu'on acidifie à une température comprise entre 0 et 5°C une solution aqueuse d'un clavulanate impur, et on extrait l'acide clavulanique dans un solvant organique non miscible à l'eau, en particulier l'acétate d'éthyle, pour obtenir une solution d'acide clavulanique dans un solvant organique, que l'on sèche ensuite et que l'on décolore par les techniques usuelles ; à la solution, dans le solvant organique décoloré et séché, d'acide clavulanique à °C on ajoute de l'octylamine tertiaire ou une solution de cette dernière dans un solvant, de façon à provoquer la formation d'un sel d'octylamine tertiaire cristallin essentiellement pur de l'acide clavulanique, qui est récupéré par les techniques usuelles ; le sel d'octylamine tertiaire intermédiaire ainsi préparé de l'acide clavulanique est dissous dans de l'isopropanol, auquel on ajoute une solution alcoolique d'éthylhexanoate de potassium, de façon à obtenir le sel cristallin souhaité, acceptable d'un point de vue pharmaceutique, de l'acide clavulanique, que l'on isole ensuite par les techniques usuelles.

2. Procédé selon la revendication 1, dans lequel le sel d'octylamine tertiaire de l'acide clavulanique est formé et utilisé en tant qu'intermédiaire de la préparation d'un clavulanate de potassium acceptable d'un point de vue pharmaceutique.
